# EUROPÄISCHE PATENTSCHRIFT

(11) **EP 1 769 279 B1**
(45) Veröffentlichungstag und Bekanntmachung des Hinweises auf die Patenterteilung: **21.04.2010**
(21) Anmeldenummer: 05770238.3
(22) Anmeldetag: 12.07.2005
(51) Int. Cl.: G02B 21/00, G02B 21/18, G02B 21/22, G02B 21/36

(54) **VORRICHTUNG ZUR BEARBEITUNG EINES OBJEKTES MITTELS LASER-STRAHLUNG**
DEVICE FOR MACHINING AN OBJECT BY MEANS OF LASER RADIATION
DISPOSITIF POUR USINER UN OBJET AU MOYEN DE RAYONS LASER

(30) Priorität: 16.07.2004 DE 102004034952; 11.07.2005 DE 102005032946
(43) Veröffentlichungstag der Anmeldung: 04.04.2007
(73) Patentinhaber: Carl Zeiss Meditec AG, 07745 Jena (DE)
(72) Erfinder: HANFT, Marco, 07749 Jena (DE); MÜHLHOFF, Dirk, 07751 Jena (DE); GERLACH, Mario, 07768 Altenberga (DE); EBERT, Elke, 07743 Jena (DE)
(74) Vertreter: Beck, Bernard
(86) Internationale Anmeldenummer: PCT/EP2005/007519
(87) Internationale Veröffentlichungsnummer: WO 2006/008025

(56) Entgegenhaltungen:
- EP-A- 0 815 801
- US-A1- 2004 047 031
- US-B1- 6 292 214
- US-B1- 6 485 413
- PATENT ABSTRACTS OF JAPAN Bd. 2000, Nr. 05, 14. September 2000 (2000-09-14) -& JP 2000 056235 A (OLYMPUS OPTICAL CO LTD), 25. Februar 2000 (2000-02-25)

## Beschreibung

### Gebiet der Ereindung

Die Erfindung bezieht sich auf eine Vorrichtung zur Bearbeitung eines Objektes mit Hilfe von Laserstrahlung, umfassend eine Beobachtungseinrichtung zur Abbildung des Objektes und eine Laser-Scan-Einrichtung, mit welcher die Laserstrahlung zwecks Energieeintrags scannend über einen vorgegebenen Sektor auf oder innerhalb des Objektes geführt wird.

### Stand der Technik

Im Stand der Technik sind Verfahren und Vorrichtungen zur Bearbeitung von Objekten mit Hilfe von Laserstrahlung bekannt, die beispielsweise zum Formen der Augenhornhaut (Kornea) zwecks Beseitigung von Fehlsichtigkeit genutzt werden. Die zur Bearbeitung erforderliche Energie wird mit gepulster Laserstrahlung in das Gewebe eingebracht, wobei die Strahlung scannend über den Bereich der Linse geführt wird, von dem Gewebe abgetragen werden soll.

Neuere Entwicklungen im Zusammenhang mit derartigen augenoptischen Operationsverfahren zur Beseitigung von Fehlsichtigkeit verwenden zum Energieeintrag Ultrakurzpulslaser und sind unter der Bezeichnung fs-LASIK bekannt. Der Entwicklungsstand hierzu ist beispielsweise beschrieben in R. Kurtz et al. "Femtosecond Laser Corneal Refractive Surgery", Proc. SPIE 3591, 209 (1999*)*.

Hierbei wird der Laserstrahl auf einen Fokuspunkt in der Größenordnung weniger Mikrometer in die Hornhaut fokussiert. Im Fokus entsteht ein Plasma, welches das unmittelbar angrenzende Gewebe schnell verdampft und dadurch eine Trennung des Gewebes an dieser Stelle bewirkt.

Diese Wechselwirkung der Laserstrahlung mit dem Gewebe wird als Photodisruption bezeichnet. Da die Photodisruption auf einen mikroskopisch kleinen Bereich beschränkt bleibt, können präzise chirurgische Schnitte innerhalb des Auges vorgenommen werden, um so eine lokal begrenzte Trennung des Hornhautgewebes zu erzielen. Durch gezielte Aneinanderreihung solcher Trennungszonen sind makroskopische Schnitte möglich, und es läßt sich ein vorgegebenes Hornhautteilvolumen isolieren. Mit der Entnahme dieses Teilvolumens wird eine gewünschte Brechkraftänderung der Hornhaut und damit eine Fehlsichtigkeitskorrektur erreicht. Die auf der Photodisruption beruhende Methode wird im folgenden als fs-LASIK bezeichnet.

Der Verfahrensablauf sieht vor, daß vor Beginn der fs-LASIK zunächst mit Hilfe eines Beobachtungsgerätes, bevorzugt eines Mikroskops, das zu bearbeitende Objekt inspiziert wird und die Parameter für den Bearbeitungsprozeß, wie Intensität der Laserstrahlung, Impulsfolgen, Länge und Verlauf der einzubringenden Schnitte u.ä. festgelegt werden. Nachfolgend wird mittels der Laser-Scan-Einrichtung die Bearbeitung vorgenommen.

In der Regel wird das Bearbeitungsergebnis wiederum mit Hilfe des Beobachtungsgerätes bewertet. Es werden nachbereitende Arbeiten, wie die Entnahme des ausgeschnittenen Korrekturvolumens und die Wundversorgung, durchgeführt.

Um die Handhabung und den Ablauf der einzelnen Verfahrensschritte optimal und effektiv gestalten zu können ist es wünschenswert, Beobachtungsgerät und Laser-Scan-Einrichtung so miteinander zu verknüpfen, daß der Operateur sowohl bei der Vorbereitung, während der Bearbeitung als auch zur Nachkontrolle stets die Blickrichtung in die Okulare des Beobachtungsgerätes beibehalten kann.

Allerdings ist die Verknüpfung von Beobachtungsgerät und Laser-Scan-Einrichtung insofern problematisch, als zunächst zur Vorbereitung eine Abbildung erzeugt werden muß, die es ermöglicht, das Objekt im Hinblick auf die nachfolgende Bearbeitung zu bewerten und den Sektor auf oder innerhalb des Objektes auszuwählen, der bearbeitet werden soll. Dazu ist es wünschenswert, einen Bereich des Objektes abzubilden, der deutlich größer ist als der zu bearbeitende Sektor. Außerdem sind während der Betrachtung durch das Beobachtungsgerät hindurch häufig auch Manipulationen mit Instrumenten am Objekt vorzunehmen, was genügend Raum zwischen dem Objektiv des Beobachtungsgerätes und dem Objekt erfordert.

Um dies zu gewährleisten, wird ein Beobachtungsgerät mit einem Abbildungssystem genutzt, das eine Schnittweite >100 mm aufweist, so daß sowohl Abbildungen genügend großer Bereiche des Objektes möglich sind und aufgrund des Abstandes zwischen Objektiv und Objekt auch Raum für Manipulationen mit Instrumenten über dem Objekt vorhanden ist.

Im Stadium der Bearbeitung dagegen ist es erforderlich, den Abstand zwischen dem Objekt und dem Objektiv, durch das der Laserstrahl auf das Objekt gerichtet wird, klein zu halten, damit das Objektiv trotz der verhältnismäßig großen Apertur und des großen Feldes in kompakter Weise ausführbar und außerdem die Überbrückung des Abstandes zwischen Objektiv und Objekt mit einem Kontaktglas möglich ist, das zur Fixierung des Objektes dient, insbesondere wenn es sich bei dem Objekt um ein Auge handelt.

Um diesen unterschiedlichen, sich in gewisser Weise widersprechenden Forderungen an die optischen Systeme für das Stadium der Vorbereitung einerseits und das Bearbeitungsstadium andererseits gerecht zu werden, sind Vorrichtungen entwickelt worden, die über ein Abbildungssystem mit variabler Schnittweite verfügen. Ein solches Abbildungssystem ist beispielsweise beschrieben in US 2003/0053219 A.

Dieses Abbildungssystem hat eine verhältnismäßig große Schnittweite, die aus den oben genannten Gründen im Vorbereitungs- und Kontrollmodus genutzt wird. Für den Bearbeitungsmodus dagegen wird die Schnittweite verringert, indem optische Elemente aus dem Abbildungsstrahlengang entfernt werden.

Nachteilig bei dieser Verfahrensweise und einem solchen Abbildungssystem ist vor allem, daß der Abstand zwischen Objektiv und Objekt im Vorbereitungs- und Kontrollmodus trotzdem nicht groß genug ist, keine stereoskopische Beobachtung möglich ist und das Objektiv bzw. die letzte, dem Objekt am nächsten gelegene Linse einen sehr großen Durchmesser aufweisen muß.

Bei einer wünschenswerten Schnittweite von beispielsweise ca. 200 mm würde der Durchmesser der letzten, objektnahen Linse etwa 45 mm betragen. Ein solcher Durchmesser erschwert aber aus anatomischen Gründen die Heranführung des Objektivs an das Objekt, da bei diesen Abmessungen das Objektiv mit der Nase oder der Stirn des Patienten während einer Augenbehandlung kollidieren kann.

*In* US-A1-200410047031 *ist eine mikroskopische Einrichtung beschrieben, die zur Beobachtung von bevorzugt biologischen Proben ausgebildet ist, wobei mit den hier angegebenen Mitteln die Probe wahlweise in einer zweidimensionalen Darstellung oder in einer dreidimensionalen Darstellung zu betrachten ist. Die Möglichkeit der Bearbeitung der Probe mittels Laserstrahlung ist nicht vorgesehen. In* US-B1-6485413 *sind verschiedene Verfahren und Anordnungen offenbart, mit denen Laserstrahlung scannend auf eine Probe gerichtet werden kann. Die Anwendung ist in therapeutischen und diagnostischen Geräten zur zielgerichteten Beleuchtung von Probenbereichen vorgesehen. Eine weitere mikroskopische Anordnung zu Beobachtung von Proben, ist in* JP-A-200056235 *offenbart. Diese Anordnung verfügt über zwei Objektive und eine Positioniereinrichtung für die Probe, wobei in einer ersten Position die Probe zunächst durch das eine der beiden Objektive und in einer veränderten zweiten Position die Probe durch das andere der beiden Objektive betrachtet werden kann. Die beiden Objektive unterscheiden sich bezüglich ihrer Vergrößerung. In* US-B1-6292214 *ist eine mikroskopische Anordnung beschrieben, die zur digitalen Photographie von Proben ausgebildet ist. Hierbei erfolgt die Übertragung von Bildsignalen innerhalb der mikroskopischen Anordnung in elektronischer Form, nachdem eine Wandlung der optischen Bildsignale in elektronische Bildsignale vorgenommen worden ist.*

### Beschreibung der Erfindung

Von diesem Stand der Technik ausgehend liegt der Erfindung die Aufgabe zugrunde, eine Einrichtung der eingangs beschriebenen Art derart weiterzuentwickeln, daß sowohl im Vorbereitungs- und Kontrollmodus als auch im Bearbeitungsmodus der zu bearbeitende Sektor in einer zur Beobachtung ergonomisch günstigeren Weise abgebildet wird.

Diese Aufgabe wird gelöst mit einer Vorrichtung zur Bearbeitung eines Objektes mit Hilfe von Laserstrahlung nach Anspruch 1, im wesentlichen umfassend
- eine Beobachtungseinrichtung mit einem ersten Objektiv zur Abbildung des Objektes und
- eine Laser-Scan-Einrichtung mit einem zweiten Objektiv, durch welches hindurch die Laserstrahlung scannend durch einen Sektor des Objektes geführt wird, der bearbeitet werden soll, wobei
- die beiden Objektive hinsichtlich der Größe der in den Abbildungen darstellbaren Bereiche und/oder hinsichtlich ihrer Schnittweite voneinander verschieden sind und
- Mittel zur wechselnden Abbildung des jeweiligen Bereiches des Objektes in einem ersten Betriebsmodus durch das erste Objektiv und in einem zweiten Betriebsmodus durch das zweite Objektiv vorhanden sind.

Die Nachteile des Standes der Technik sind damit behoben, weil es nun möglich ist, in den beiden Betriebsmodi jeweils ein gesondertes, dem jeweiligen Anliegen angepaßtes Objektiv zur Abbildung zu nutzen, nämlich einerseits im ersten Betriebsmodus, der im folgenden als Vorbereitungs- und Kontrollmodus bezeichnet wird, eine Abbildungsoptik mit großer Schnittweite zur bevorzugt stereoskopischen Abbildung eines Bereiches des Objektes, der mindestens ebenso groß ist wie der zu bearbeitende Sektor, und andererseits im zweiten Betriebsmodus, nachfolgend als Bearbeitungsmodus bezeichnet, eine Abbildungsoptik mit kleiner Schnittweite ohne Behinderung nahe an das Objekt herangebracht werden kann und sowohl zum Scannen des Laserstrahls als auch zur Abbildung des bearbeiteten Sektors während der Bearbeitung geeignet ist.

Bevorzugt ist die erfindungsgemäße Vorrichtung mit einer Positioniereinrichtung ausgestattet, um die Position des Objektes relativ zu den beiden Objektiven verändern zu können, wobei sich im Vorbereitungs- und Kontrollmodus das Objekt im Fokus des ersten Objektivs und im Bearbeitungsmodus das Objekt im Fokus des zweiten Objektivs befindet.

Damit ist in der Position der Vorbereitung ein genügend großer Arbeitsabstand zwischen Objektiv und Objekt vorhanden, während in der anderen Position der Abstand deutlich reduziert ist.

Ein wesentlicher Erfindungsgedanke besteht demzufolge darin, die Laser-Scan-Einrichtung und die Beobachtungseinrichtung miteinander zu koppeln, indem das Objektiv der Beobachtungseinrichtung für die Zeit der Bearbeitung ersetzt wird durch das Objektiv der Laser-Scan-Einrichtung, wobei die Scanfunktion des Objektivs der Laser-Scan-Einrichtung erhalten bleibt, dieses jedoch zusätzlich zur Beobachtung genutzt wird.

Um dies zu ermöglichen, ist im Strahlengang der Laser-Scan-Einrichtung eine Auskoppeloptik für das während der Bearbeitung vom Objekt kommende und durch das Objektiv der Laser-Scan-Einrichtung hindurchtretende Licht vorhanden, und im Strahlengang der Beobachtungseinrichtung ist eine mit dieser Auskoppeloptik korrespondierende Einkoppeloptik vorgesehen, so daß das Objektiv der Laser-Scan-Einrichtung während des Bearbeitungsmodus anstelle des zur Beobachtung im Vorbereitungs- und Kontrollmodus dienenden Objektivs der Beobachtungseinrichtung genutzt wird.

Zur Übertragung des Lichts von der Auskoppeloptik zur Einkoppeloptik sind optische und/oder opto-elektronische Baugruppen vorhanden. Vorteilhaft ist es, wenn im Lichtweg zwischen dem Objektiv der Beobachtungseinrichtung und der Einkoppeloptik ein ansteuerbarer Shutter vorgesehen ist, mit dem das im Bearbeitungsmodus vom ungenutzten Objektiv der Beobachtungseinrichtung kommende Licht abgeblockt werden kann.

Außerdem sollte im Lichtweg zwischen dem Objektiv der Laser-Scan-Einrichtung und der Einkoppeloptik ein Shutter vorgesehen sein, der im Vorbereitungs- und Kontrollmodus das durch das hierbei ungenutzte Objektiv der Laser-Scan-Einrichtung kommende Licht abblockt.

So wird im jeweiligen Betriebsmodus eine unerwünschte Beeinflussung der Abbildungen durch Falschlicht vermieden.

In einer besonders bevorzugten Ausgestaltung der erfindungsgemäßen Vorrichtung ist die Beobachtungseinrichtung als Stereomikroskop ausgebildet und umfaßt im wesentlichen
- das Stereomikroskopobjektiv als erstes Objektiv,
- zwei getrennte, eine stereoskopische Darstellung ermöglichende Abbildungsstrahlengänge,
- in jedem der beiden Abbildungsstrahlengängen jeweils einen Vergrößerungswechsler zur Vorgabe unterschiedlicher Abbildungsmaßstäbe, ein Tubuslinsensystem und ein Okular, und
- eine Einkoppeloptik.

Die Einkoppeloptik kann prinzipiell aus einem oder aus zwei Strahlteilern bestehen, die als Strahlteilerwürfel oder als Strahlteilerplatten ausgeführt und so ausgelegt sind, daß Licht jeweils aus zwei Richtungen eingekoppelt und in zwei Richtungen ausgekoppelt werden kann. Auf diese Weise ist neben der Einspiegelung des Bildes in den Abbildungsstrahlengang zur visuellen Beobachtung auch die teilweise Lichtauskopplung für ein weiteres Beobachtungsgerät, z.B. einer CCD-Kamera, möglich.

Mit einer CCD-Kamera oder einer anderen bildgebenden Einrichtung ist neben der reinen Beobachtungsfunktion auch die Basis für weitere Funktionen geschaffen. So kann mit den damit gewonnenen Abbildungen beispielsweise ein Justiervorgang für den Bearbeitungsmodus unterstützt werden. Bei der Zielsetzung eine Regelschleife einzurichten, bildet eine CCD-Kamera in Kombination mit einem Computer und einem geeigneten Manipulator einen wesentlichen Baustein.

Vorzugsweise besteht die Einkoppeloptik aus zwei Strahlteilern, wobei je ein Strahlteiler einem der beiden Abbildungsstrahlengänge zugeordnet und dort jeweils zwischen dem Vergrößerungswechsler und dem Tubuslinsensystem angeordnet ist. Durch Auswahl der Teilungsverhältnisse (Verhältnis Transmission/Reflexion) an den Teilerschichten der Strahlteiler kann die Helligkeiten der Abbildungen variiert werden.

Es ist empfehlenswert, zwischen den beiden Strahlteilern einen Shutter vorzusehen, um eine störende gegenseitige Beeinflussung der beiden Abbildungsstrahlengänge durch das eingekoppelte Licht zu vermeiden.

Im Rahmen der Erfindung liegt es selbstverständlich auch, wenn anstelle der stereoskopischen Beobachtungseinrichtung ein optisches Beobachtungssystem vorgesehen ist, mit dem eine monoskopische Beobachtung erfolgt.

Zur Übertragung des Lichts von der Laser-Scan-Einrichtung zur Beobachtungseinrichtung können optische Baugruppen aus Linsen, Prismen, Strahlumlenkelementen und/oder faseroptischen Lichtleitern vorgesehen sein.

Damit wird das vom Objekt ausgehende und durch das Objektiv der Laser-Scan-Einrichtung hindurchtretende Licht zur Beobachtungseinrichtung übertragen und dort mittels der Einkoppeloptik in die Abbildungsstrahlengänge eingekoppelt.

In konkreter Ausgestaltung können die optischen Baugruppen zur Übertragung des Lichts von der Laser-Scan-Einrichtung zur Beobachtungseinrichtung im wesentlichen umfassen:
- eine Linsengruppe mit nachgeordneter Feldlinse zur Erzeugung eines Zwischenbildes aus dem durch das zweite Objektiv, nämlich dem Objektiv der Laser-Scan-Einrichtung, kommenden Lichtbündel,
- eine Linsengruppe zur Abbildung des Zwischenbildes in mindestens einen der beiden Abbildungsstrahlengänge der Beobachtungseinrichtung, sowie
- optische Elemente zur Strahlumlenkung und/oder Strahlfaltung.

Zusätzlich kann in den Übertragungsweg des Lichtes von der Laser-Scan-Einrichtung zur Beobachtungseinrichtung ein optisches Zoomsystem zur Variation der Brennweite bzw. des Abbildungsmaßstabes für das Zwischenbild eingeordnet sein, das beispielsweise zwei in ihrer Position zueinander veränderbare Linsen oder Linsengruppen umfaßt.

Damit hat der Operateur die Möglichkeit, den zu bearbeitenden und dabei zu beobachtenden Sektor nach Bedarf größer oder zu kleiner abzubilden.

Im Rahmen der Erfindung liegt es weiterhin, zur Übertragung des Lichts von der Laser-Scan-Einrichtung zur Beobachtungseinrichtung opto-elektronische Baugruppen vorzusehen. So kann beispielsweise im Bearbeitungsmodus die Abbildung des Objektes mittels des zweiten Objektivs, also des Objektivs der Laser-Scan-Einrichtung, in eine opto-elektronische Kamera erfolgen, die im wesentlichen aus einem Videoobjektiv und einem CCD-Empfänger besteht. Der Kamera sind dann eine Bildsignalverarbeitungseinrichtung und eine Bildwiedergabeeinrichtung nachgeordnet, und es sind Mittel zur Einkopplung des auf der Bildwiedergabeeinrichtung wiedergegebenen Bildes in mindestens einen der beiden Abbildungsstrahlengänge der Beobachtungseinrichtung vorgesehen, so daß auch auf diese Weise im Bearbeitungsmodus der zu bearbeitende Sektor durch die Okulare bzw. das Okular der Beobachtungseinrichtung hindurch betrachtet werden kann.

Als Bildwiedergabeeinrichtung kann dabei vorteilhaft ein LC-Display zur Anwendung kommen.

Denkbar ist es weiterhin, zur Übertragung des Lichts von der Laser-Scan-Einrichtung zur Beobachtungseinrichtung sowohl optische als auch opto-elektronische Baugruppen miteinander kombiniert einzusetzen oder auch wahlweise gegeneinander austauschbar zu nutzen.

In einer weiteren, speziellen Ausgestaltung ist die erfindungsgemäße Vorrichtung ergänzt mit einer Anordnung, die es ermöglicht, die Bearbeitung betreffende Prozeßparameter zu ermitteln, Werte zu ermitteln, welche die Eigenschaften des Materials charakterisieren, aus dem das Objekt besteht und Meßwerte zu erfassen, die Auskunft über die Ausdehnung des Objektes oder den zu bearbeitenden Sektor geben.

Diesbezüglich können des weiteren Mittel zur Einspiegelung der so gewonnenen Informationen in einen oder beide Abbildungsstrahlengänge der Beobachtungseinrichtung vorhanden sein, so daß das Bedienungspersonal während des Bearbeitungsmodus durch das Okular bzw. die Okulare hindurch sowohl den Sektor, der bearbeitet wird, beobachten kann und zugleich auch die Informationen über Prozeßparameter, Abmessungen und/oder Materialeigenschaften erhält.

Im Zusammenhang damit besteht eine weitere besondere Ausgestaltungsmöglichkeit darin, die Beobachtungseinrichtung als Stereomikroskop nach dem Greenough-Typ auszubilden. Bei diesem Mikroskop ist in jedem der beiden stereoskopischen Abbildungsstrahlengänge ein Objektiv vorhanden und es besteht daher vorteilhaft die Möglichkeit, die Einrichtung zur Ermittlung von Informationen über Prozeßparameter, Objektabmessungen oder Materialeigenschaften im Raum zwischen diesen beiden Objektiven anzuordnen.

### Kurze Beschreibung der Zeichungen

Die Erfindung soll nachfolgend anhand von Ausführungsbeispielen näher erläutert werden. In den zugehörigen Zeichnungen zeigen
- Fig.1: die Prinzipdarstellung einer Vorrichtung zur Bearbeitung eines Objektes mit Laserstrahlung nach Stand der Technik im Stadium der Vorbereitung,
- Fig.2: die Prinzipdarstellung der Vorrichtung nach Fig.1 im Stadium der Bearbeitung,
- Fig.3: eine Prinzipdarstellung der erfindungsgemäßen Vorrichtung zur Bearbeitung eines Objektes mit Laserstrahlung im Vorbereitungs- und Kontrollmodus, ausgestattet mit optischen Baugruppen zur Auskopplung von Licht aus der Laser-Scan-Einrichtung und zur Einkopplung in die Abbildungsstrahlengänge einer als Stereomikroskop ausgebildeten Beobachtungseinrichtung,
- Fig.4: die Prinzipdarstellung der erfindungsgemäßen Vorrichtung nach Fig.3 im Bearbeitungsmodus,
- Fig.5: die erfindungsgemäße Vorrichtung mit weiteren optischen Baugruppen zur Auskopplung von Licht aus der Laser-Scan-Einrichtung und zu dessen Einkopplung in die Abbildungsstrahlengänge der als Stereomikroskop ausgebildeten Beobachtungseinrichtung,
- Fig.6: die erfindungsgemäße Vorrichtung mit optoelektronischen Baugruppen zur Auskopplung von Licht aus der Laser-Scan-Einrichtung und zu dessen Einkopplung in die Abbildungsstrahlengänge der Beobachtungseinrichtung,
- Fig.7: eine Ausgestaltung der erfindungsgemäßen Vorrichtung, bei der die Beobachtungseinrichtung als Stereomikroskop nach dem Greenough-Typ ausgebildet ist und zwischen den Frontobjektiven dieses Stereomikroskops eine Einrichtung zur Erfassung von Prozeßparametern, Materialeigenschaften und/oder Objektabmessungen eingeordnet ist,
- Fig.8: eine konkretisierte Ausführung optischer Baugruppen und deren Zusammenwirken bei der Lichtübertragung von der Laser-Scan-Einrichtung zur Beobachtungseinrichtung,
- Fig.9: eine Ausgestaltung der erfindungsgemäßen Vorrichtung, bei der die Einkoppeloptik als Binokularteiler mit Auskopplung eines Abbildungsstrahlengangs für eine CCD-Kamera ausgebildet ist,
- Fig.10: die beispielhafte Ausführung des Binokularteilers aus Fig.9 einschließlich dreier bevorzugter Vorgaben für Teilungsverhältnisse,
- Fig.11: beispielhaft die Anordnung von Zielmarken, die zur Ausrichtung des zu bearbeitenden Objektes relativ zur Laser-Scan-Einrichtung dienen, in Zwischenbildebenen der Vorrichtung.

### Ausführliche Beschreibung der Zeichnungen

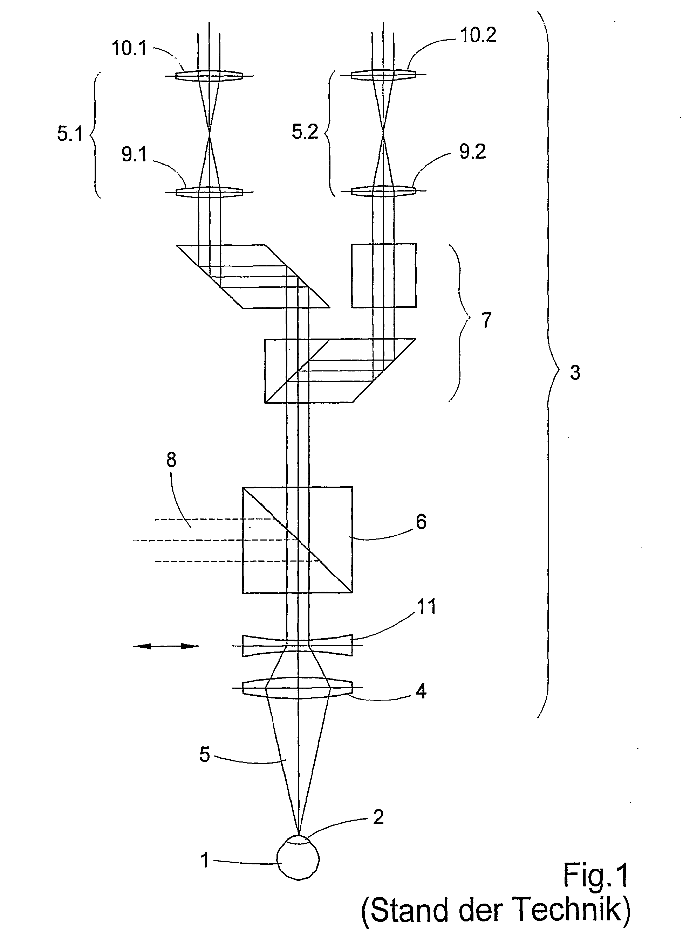

In Fig.1 sind die wesentlichsten Baugruppen einer Vorrichtung zur Bearbeitung eines Objektes 1 nach der fs-LASIK dargestellt, wie sie im Stand der Technik bekannt ist.

Bei dem Objekt 1 handelt es sich um das Auge eines Patienten, von dem ein Sektor 2, der unter der Oberfläche liegen oder auch an die Oberfläche angrenzen kann, durch Photodisruption bearbeitet werden soll.

Zur Bearbeitung wird eine Laserquelle genutzt, die eine Strahlung mit ultrakurzen Pulsen erzeugt und der eine Laser-Scan-Einrichtung nachgeordnet ist. Entsprechende Einrichtungen sind aus der eingangs angegebenen Literaturstelle wie auch aus anderen Dokumentationen bekannt, so daß sich an dieser Stelle eine ausführliche Erläuterung des Verfahrens der Photodisruption erübrigt.

Die Vorrichtung nach Stand der Technik befindet sich in Fig.1 im Stadium der Vorbereitung der Bearbeitung und kann in dieser Konfiguration auch zur Kontrolle des Ergebnisses nach der Bearbeitung genutzt werden.

Eine Beobachtungseinrichtung 3, beispielsweise ein Mikroskop, dient vor Beginn des Bearbeitungsprozesses zunächst dazu, einen Ausschnitt des Objektes 1 mehr oder weniger groß abzubilden, der den Sektor 2 umfaßt, der nachfolgend bearbeitet werden soll, so daß der Operateur die Möglichkeit hat, den Sektor 2 lokal einzugrenzen und die Bearbeitungsparameter vorzugeben, wie Strahlungsintensität, Dauer der Bestrahlung, Pulsfolge und -länge und ähnlich.

Die Beobachtungseinrichtung 3 weist ein Objektiv 4 auf, durch welches das vom Objekt 1 kommende Licht in einem Strahlengang 5 hindurchtritt.

Dem Objektiv 4 sind ein Strahlteiler 6 und ein Teilersystem 7 nachgeordnet. Über den Strahlteiler 6 kann die zur Bearbeitung dienende Laserstrahlung 8 eingekoppelt werden; mit dem Teilersystem 7 erfolgt die Aufzweigung des Strahlenganges 5 in zwei Teilstrahlengänge 5.1 und 5.2. In den Teilstrahlengängen 5.1, 5.2 sind Tubuslinsen 9.1, 9.2 und Okulare 10.1, 10.2 vorgesehen.

Mittels einer in den Strahlengang 5 ein- und ausschwenkbaren Linsengruppe 11 kann die Schnittweite des optischen Systems, bestehend aus Objektiv 4 und Linsengruppe 11, verändert werden, wie dies in US 2003/0053219 A1 beschrieben ist. Ist die Linsengruppe 11, wie in Fig.1 dargestellt, in den Strahlengang 5 eingeschwenkt, erfolgt die Abbildung des Objektes 1 mit einer Schnittweite von beispielsweise 100 mm. Das hat den Vorteil, daß erstens große Bereiche des zu behandelnden Objektes 1 abgebildet und beobachtet werden können, und zweitens zwischen Objekt 1 und Objektiv 4 Raum zur Manipulation mit Instrumenten vorhanden ist.

Die Laserstrahlungsquelle und die Laser-Scan-Einrichtung sind in dem in Fig.1 gezeigten Stadium der Vorbereitung des Bearbeitungsprozesses nicht in Betrieb. Zur Verdeutlichung dessen sind in Fig.1, wie auch in den weiteren Zeichnungen, aktive Strahlengänge durch volle Linien, aktuell nicht genutzte Strahlengänge durch unterbrochene Linien dargestellt.

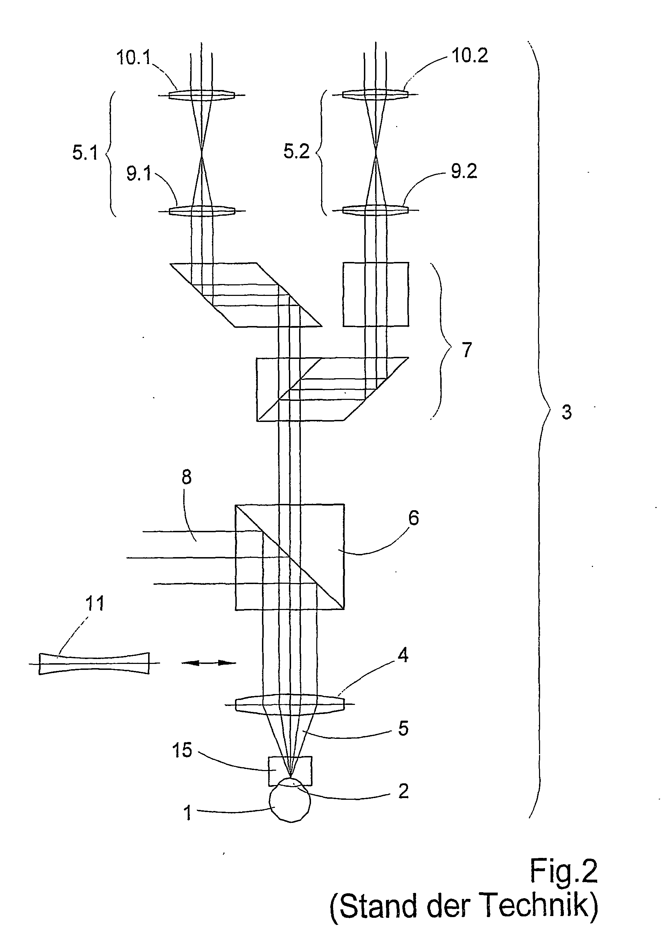

Ist die Vorbereitung des Bearbeitungsprozesses abgeschlossen, wird die Vorrichtung in den Bearbeitungsmodus umgestellt, der in Fig.2 gezeigt ist.

Zur Umstellung wird zunächst die Linsengruppe 11 aus dem Strahlengang 5 entfernt, wodurch sich die Schnittweite der Abbildungsoptik verringert. Die geringere Schnittweite im Bearbeitungsmodus ist erforderlich, um den Fokusabstand zwischen Objektiv 4 und Objekt 1 klein zu halten, damit der Zwischenraum, der jetzt nicht mehr für Manipulationen mit Instrumenten benötigt wird, mit einem Kontaktglas überbrückt werden kann, das zur Fixierung des Objektes 1 notwendig ist.

Anschließend werden Laserquelle und Laser-Scan-Einrichtung in Betrieb genommen, die Laserstrahlung 8 über den Strahlteiler 6 in den Strahlengang 5 eingekoppelt, durch das Objektiv 4 hindurch auf das Objekt 1 gerichtet und dabei scannend durch oder über den ausgewählten Sektor 2 bewegt, in dem nun die Photodisruption stattfindet. Der Sektor 2 und der Verlauf des Bearbeitungsprozesses werden mittels der Beobachtungseinrichtung 3 visualisiert.

Ein wesentlicher Nachteil an dem hier dargestellten Stand der Technik besteht darin, daß die Beobachtung nur anhand einer monoskopischen Abbildung möglich ist.

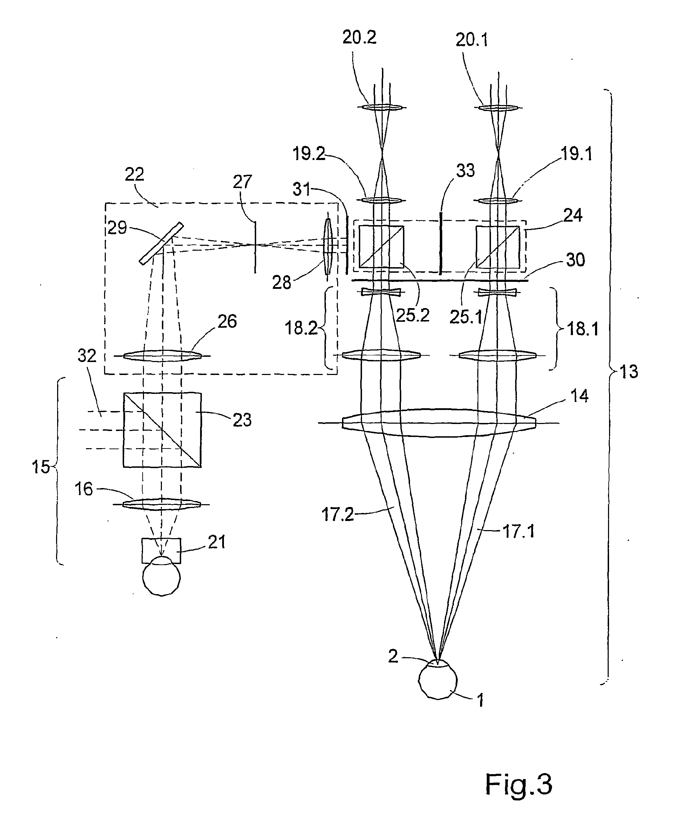

Um zu erreichen, daß sowohl im Vorbereitungsmodus als auch im Bearbeitungsmodus nur derselbe Tubuseinblick (Okulare 20.1, 20.2 und Tubuslinsensysteme 19.1, 19.2) genutzt werden kann, eine stereoskopische Beobachtung im Vorbereitungsmodus möglich ist und außerdem auch die Bedingung erfüllt ist, daß im Vorbereitungsmodus (und auch im Kontrollmodus) die Abbildung mit einer großen und im Bearbeitungsmodus mit einer kleinen Schnittweite erfolgt, wurde der in Fig.1 und Fig.2 dargestellte Stand der Technik mit der erfindungsgemäßen Vorrichtung weiterentwickelt, die nachfolgend anhand Fig.3 bis Fig.8 erläutert wird.

Wie in Fig.3 im Prinzip dargestellt, umfaßt die erfindungsgemäße Vorrichtung eine Beobachtungseinrichtung 13 mit einem ersten Objektiv 14 und eine Laser-Scan-Einrichtung 15 mit einem zweiten Objektiv 16. Die Beobachtungseinrichtung 13 ist als Stereomikroskop ausgebildet und weist in zwei Abbildungsstrahlengängen 17.1 und 17.2 jeweils einen Vergrößerungswechsler 18.1 und 18.2, ein Tubuslinsensystem 19.1 und 19.2 sowie ein Okular 20.1 und 20.2 auf.

Durch die Okulare 20.1 und 20.2 hindurch kann der Operateur je nach Einstellung des Abbildungsmaßstabes mittels der Vergrößerungswechsler 18.1, 18.2 einen mehr oder weniger großen Bereich des Objektes 1 betrachten, aus diesem Bereich den zu bearbeitenden Sektor 2 auswählen und aufgrund seiner Erfahrungen und Kenntnisse in Abschätzung der Gegebenheiten die Bearbeitungsparameter festlegen.

Wie in Fig.3 angedeutet, weist das Objektiv 14 eine verhältnismäßig große Schnittweite auf, beispielhaft größer 100 mm.

Das Objektiv 16 der Laser-Scan-Einrichtung 15 dagegen ist mit wesentlich geringerer Schnittweite, beispielsweise 5 mm, ausgeführt, so daß der Durchmesser dieses Objektivs genügend klein ausgeführt werden kann und der Zwischenraum zwischen Objekt 1 und Objektiv 16 so gering ist, daß er weitestgehend durch ein Kontaktglas 21 überbrückt werden kann.

Erfindungsgemäß ist vorgesehen, daß die Beobachtungseinrichtung 13 und die Laser-Scan-Einrichtung 15 über eine optische Koppeleinrichtung 22, die zur Übertragung des vom Objekt 1 kommenden und durch das Objektiv 16 hindurchtretenden Lichts zur Beobachtungseinrichtung 13 dient, miteinander verbunden sind.

Zur Auskopplung des zu übertragenden Lichtes aus der Laser-Scan-Einrichtung 15 ist diese mit einer Auskoppeloptik 23 ausgestattet, die beispielhaft als Strahlteiler ausgeführt ist.

Hierzu korrespondierend ist in der Beobachtungseinrichtung 13 eine Einkoppeloptik 24 vorgesehen, die vorzugsweise zwischen den Vergrößerungswechslern 18.1 und 18.2 und den Tubuslinsensystemen 19.1 und 19.2 eingeordnet ist. Die Einkoppeloptik 24 ist beispielhaft ebenfalls in Form von Strahlteilern ausgeführt, wobei ein Strahlteiler 25.1 dem Abbildungsstrahlengang 17.1 und ein weiterer Strahlteiler 25.2 dem Abbildungsstrahlengang 17.2 zugeordnet ist.

Die Koppeleinrichtung 22 ist in verschiedenen Ausgestaltungsvarianten denkbar. In der in Fig.3 dargestellten Variante besteht die Koppeleinrichtung 22 aus einer Linsengruppe 26 zur Erzeugung eines Zwischenbildes 27 und einer Linsengruppe 28 zur Abbildung dieses Zwischenbildes 27 in die Abbildungsstrahlengänge 17.1 und/oder 17.2. Ein Umlenkelement 29 dient zur Richtungsänderung des zwischen der Linsengruppe 26 und der Linsengruppe 28 verlaufenden Strahlenganges.

Wie aus Fig.3 weiterhin ersichtlich, ist im Lichtweg der Abbildungsstrahlengänge 17.1 und 17.2 ein Shutter 30 und im Lichtweg zwischen der Laser-Scan-Einrichtung 15 und der Beobachtungseinrichtung 13 ein Shutter 31 vorgesehen, die beide abwechselnd angesteuert werden können, um einmal den Lichtweg durch das Objektiv 14 zu den Okularen 20.1 und 20.2 zu blockieren (Shutter 30) oder den Lichtweg vom Objektiv 16 zu den Okularen 20.1 und 20.2 (Shutter 31) zu sperren.

In Fig.3 ist die erfindungsgemäße Vorrichtung im Vorbereitungs- und Kontrollmodus dargestellt. Das Objekt 1 befindet sich hierbei im Fokus des Objektivs 14, der Shutter 30 ist geöffnet und der Operateur kann durch die Okulare 20.1 und 20.2 hindurch das Objekt 1 beobachten und den Bearbeitungsprozeß wie oben bereits beschrieben vorbereiten.

Um bei dieser Betriebsweise ein Übersprechen bzw. eine unerwünschte gegenseitige Beeinflussung der Abbildungsstrahlengänge 17.1, 17.2 zu vermeiden, ist zwischen den Strahlteilern 25.1 und 25.2 ein weiterer Shutter 33 vorgesehen, der bei Bedarf blockierend genutzt werden kann.

Die Laserstrahlungsquelle und Laser-Scan-Einrichtung (in Fig.3 nicht dargestellt) sind außer Betrieb, was durch die unterbrochenen Linien der Strahlführung in der Laser-Scan-Einrichtung 15 dokumentiert ist. Der Shutter 31 ist geschlossen, so daß kein Licht von der Laser-Scan-Einrichtung 15 über die optische Koppeleinrichtung 22 in die Abbildungsstrahlengänge 17.1, 17.2 der Beobachtungseinrichtung 13 übertragen wird.

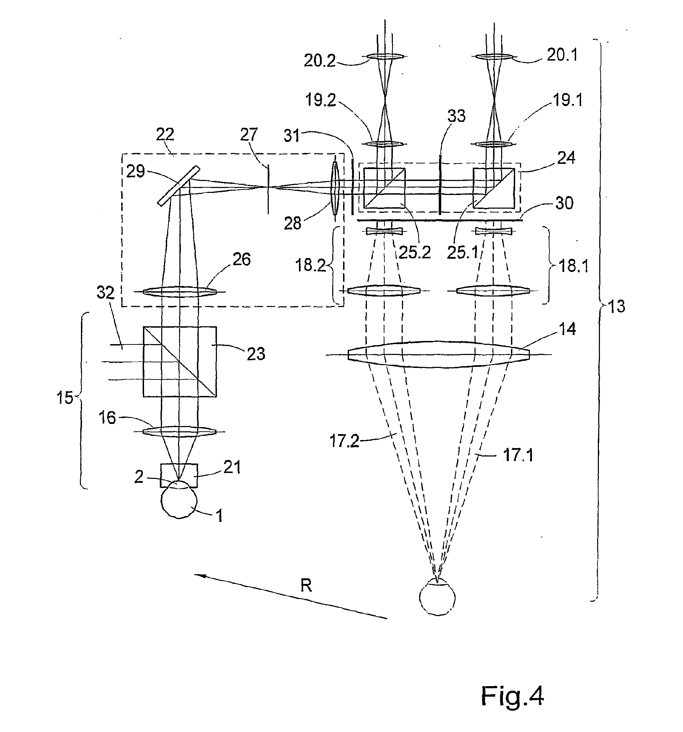

Ist die Vorbereitung des Bearbeitungsprozesses abgeschlossen und sind die Bearbeitungsparameter festgelegt, wird die Vorrichtung vom Vorbereitungs- und Kontrollmodus in den Bearbeitungsmodus umgestellt, der in Fig.4 dargestellt ist.

Dazu wird zunächst eine Verschiebung des Objektes 1 relativ zu den Objektiven 14, 16 in Richtung R vorgenommen, so daß sich das Objekt 1 nun nicht mehr im Fokus des Objektivs 14, sondern im Fokus des Objektivs 16 befindet. Die Verschiebung kann in der Weise erfolgen, daß die Vorrichtung relativ zu dem Objekt 1 bewegt wird, bevorzugt wird jedoch das Objekt 1 (bzw. der Patient) relativ zu der Vorrichtung bzw. den Objektiven 14, 16 verschoben.

Um dies zu bewerkstelligen kann eine Positioniereinrichtung vorgesehen sein, bei der sich in einer ersten Endlage das Objekt 1 im Fokus des Objektivs 14 und in einer zweiten Endlage das Objekt 1 im Fokus des Objektivs 16 befindet. Die Positioniereinrichtung ist zeichnerisch nicht dargestellt. Sie kann mit einem beweglichen Tisch ausgestattet sein, der mit einem Antrieb gekoppelt ist. Das Erreichen der jeweiligen Endlage wird durch entsprechend angeordnete Sensoren kontrolliert, die über eine Ansteuereinrichtung zur Ein- und Ausschaltung mit dem Antrieb gekoppelt sind.

Sobald das Objekt 1 das Gesichtsfeld des Objektivs 14 verlassen hat, wird durch entsprechende Ansteuerung der Shutter 30 geschlossen und der Shutter 31 geöffnet. Das Licht, das vom Objekt 1 kommend durch das Objektiv 16 hindurchtritt, erreicht nun über die Auskoppeloptik 23, die Linsengruppe 26 und die Linsengruppe 28 die Abbildungsstrahlengänge 17.1, 17.2 der Beobachtungseinrichtung 13, so daß das damit erzeugte Zwischenbild 27, umgelenkt an den Teilerflächen der Strahlteiler 25.1 und 25.2, durch die Tubuslinsensysteme 19.1 und 19.2 hindurch zu den Okularen 20.1 und 20.2 gelangt und der Operateur nunmehr das Objekt 1 bzw. den zu bearbeitenden Sektor 2 durch das Objektiv 16 betrachtet.

Zur Einleitung des Bearbeitungsprozesses wird nun die Laser-Scan-Einrichtung 15 in Betrieb genommen und die damit erzeugte Laserstrahlung 32 an der Teilerschicht der als Strahlteilers ausgeführten Auskoppeleinrichtung 23 zum Objektiv 16 umgelenkt und durch dieses hindurch scannend über bzw. durch den Sektor 2 des Objektes 1 geführt. Der Verlauf des Bearbeitungsprozesses wird vom Operateur durch das Objektiv 16 hindurch verfolgt.

Mit der erfindungsgemäßen Vorrichtung wird erreicht, daß das Objekt 1 im Vorbereitungs- und Kontrollmodus stereoskopisch beobachtet werden kann und der Operateur durch dieselben Okulare 20.1, 20.2, die der stereoskopischen Beobachtung während der Vorbereitung oder Kontrolle dienen, auch den Verlauf des Bearbeitungsprozesses verfolgen kann.

Ist die Relativverschiebung zwischen Objekt 1 und erfindungsgemäßer Vorrichtung so ausgelegt, daß die Vorrichtung in Ruhe verharrt, während das Objekt 1 in Richtung R verschoben wird, so kann der Operateur seine Blickrichtung in die Okulare 20.1, 20.2 auch beim Übergang vom Vorbereitungs- und Kontrollmodus zum Bearbeitungsmodus und umgekehrt beibehalten.

Mit dieser Vorrichtung ist es möglich, in den beiden Betriebsmodi jeweils ein dem speziellen Anliegen angepaßtes Objektiv 14 bzw. 16 zur Abbildung des Objektes 1 zu nutzen, also einerseits im Vorbereitungs- und Kontrollmodus eine stereoskopische Abbildung bei großer Schnittweite, und andererseits im Bearbeitungsmodus eine Abbildung mit kleinerer Schnittweite zu erzielen, so daß im Vorbereitungs- und Kontrollmodus vorteilhaft ein großer Abstand zwischen dem jeweiligen Objektiv und dem Objekt 1 eingehalten wird und im Bearbeitungsmodus ein geringer Abstand möglich ist, der durch das Kontaktglas 15 überbrückt werden kann.

Wie bereits dargelegt, kann die Koppeleinrichtung 22 in unterschiedlicher Weise ausgestaltet sein.

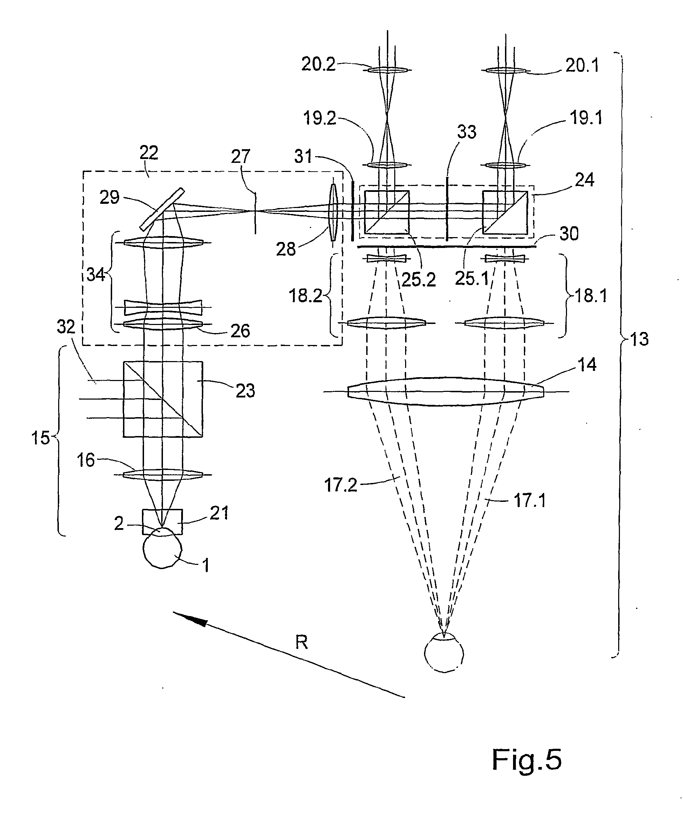

So ist in einer Ausgestaltungsvariante nach Fig.5 in den Strahlengang zwischen der Auskoppeloptik 23 und der Einkoppeloptik 24 zusätzlich ein optisches System 34 mit variabler Brennweite eingeordnet, das zur Veränderung des Abbildungsmaßstabes bei der Darstellung des Sektors 2 während des Bearbeitungsmodus dient.

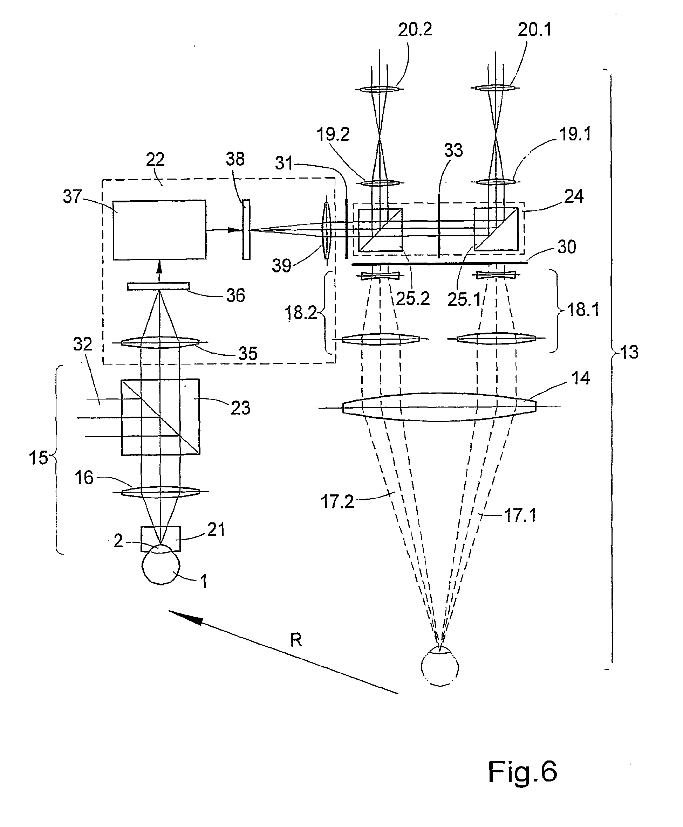

Außer den rein optischen Mitteln zur Übertragung des Lichts von der Laser-Scan-Einrichtung 15 zur Beobachtungseinrichtung 13 kann auch die Verwendung opto-elektronischer Baugruppen vorgesehen sein, wie dies in Fig.6 dargestellt ist.

Hierbei werden das Objekt 1 bzw. der Sektor 2 zunächst mit einem Videoobjektiv 35 auf einen CCD-Empfänger 36 abgebildet. Videoobjektiv 35 und CCD-Empfänger 36 sind in diesem Fall Baugruppen einer opto-elektronischen Kamera.

Wie in Fig.6 weiterhin symbolisch angedeutet, sind dem CCD-Empfänger 36 eine Bildsignalverarbeitungseinrichtung 37 sowie eine Bildwiedergabeeinrichtung 38 nachgeordnet. Die Bildwiedergabeeinrichtung 38 kann beispielsweise als LC-Display ausgebildet sein.

Die auf der Bildwiedergabeeinrichtung 38 erzeugte Abbildung wird über eine Linsengruppe 39 und die Einkoppeloptik 24, die wie bereits dargelegt vorteilhaft aus zwei Strahlteilern 25.1 und 25.2 bestehen kann, in die Abbildungsstrahlengänge 17.1, 17.2 eingekoppelt.

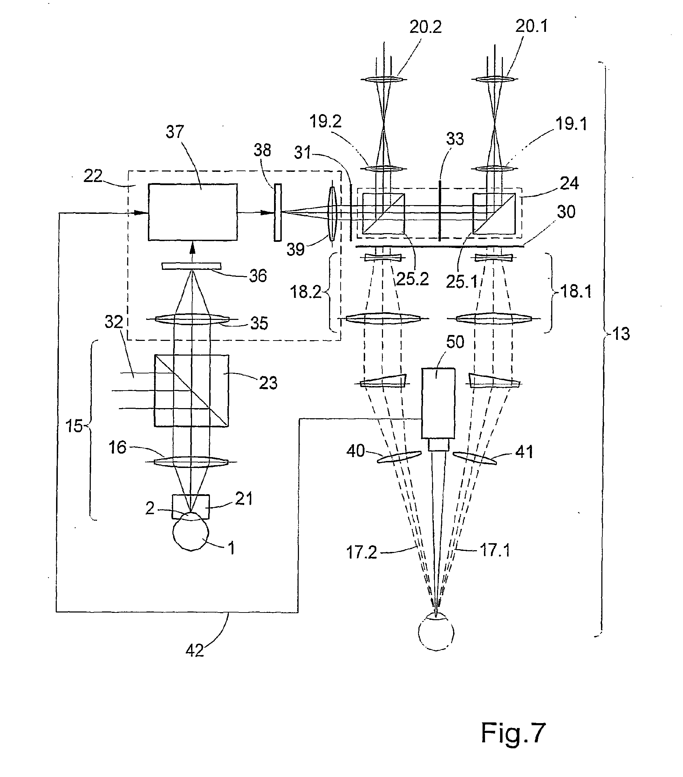

In einer Ausgestaltung der erfindungsgemäßen Vorrichtung, die in Fig.7 dargestellt ist, ist als Beobachtungseinrichtung 13 ein Stereomikroskop nach dem Greenough-Typ vorgesehen. Ein solches Stereomikroskop weist in jedem der beiden gegeneinander geneigten Beobachtungsstrahlengänge ein Frontobjektiv 40 bzw. 41 auf.

Aufgrund der Neigung gegeneinander ergibt sich zwischen den beiden Abbildungsstrahlengängen 17.1, 17.2 ein unverbauter Raum, der erfindungsgemäß zur Unterbringung einer Einrichtung 50 genutzt wird, die ausgebildet ist zur Erfassung
- von Prozeßparametern,
- von Meßwerten, welche die Eigenschaften des Materials charakterisieren, aus dem das Objekt 1 besteht, und/oder
- von Längenmeßwerten, die zur Ermittlung von Größenangaben, den Sektor 2 betreffend.

Die so zunächst optisch erfaßten Informationen werden in elektronische Signale gewandelt, über einen Signalweg 42 an die für diese Ausgestaltung entsprechend modifizierte Bildsignalverarbeitungseinrichtung 37 weitergeleitet, mittels der Bildwiedergabeeinrichtung 38 in visuell wahrnehmbare Informationen gewandelt und über die Einkoppeloptik 24 in einen oder beide Abbildungsstrahlengänge 17.1, 17.2 eingekoppelt.

Dem Operateur stehen damit in beiden Betriebsmodi nicht nur die mit den Objektiven 14 und 16 gewonnenen Abbildungen zur Verfügung, sondern beispielsweise auch in diese Abbildungen eingeblendete α-numerische Informationen.

Die Erfassung und Einkopplung dieser Informationen kann auch allein ohne Abbildung des Objektes 1 erfolgen.

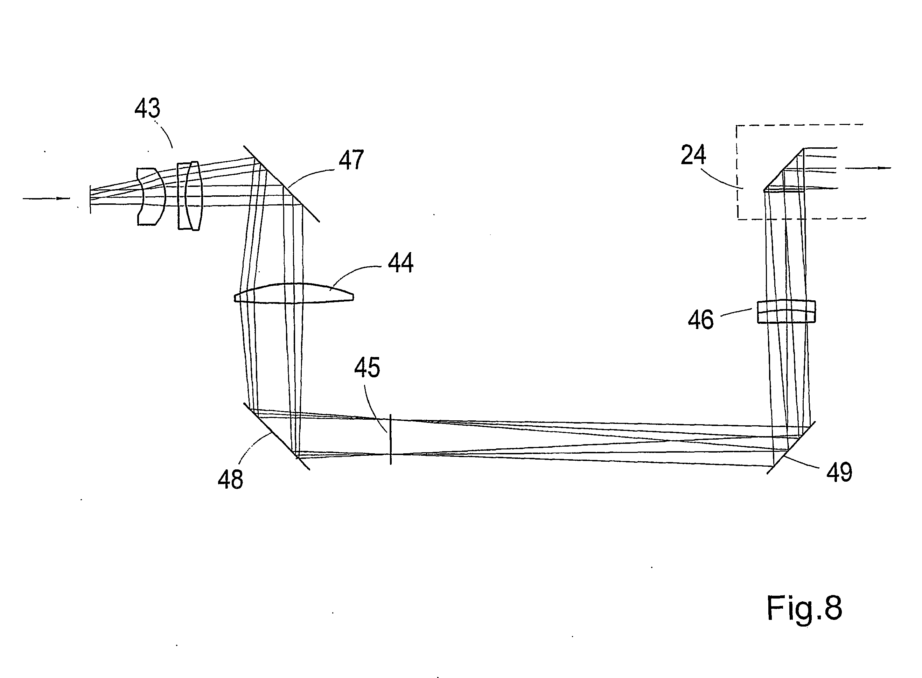

In Fig.8 ist eine konkretisierte Ausführung optischer Baugruppen und ihres Zusammenwirkens zur Lichtübertragung von der Laser-Scan-Einrichtung 15 zur Beobachtungseinrichtung 13 dargestellt, im wesentlichen umfassend
- eine optische Baugruppe 43 und eine Feldlinse 44, welche die vom Objektiv 16 der Laser-Scan-Einrichtung (hier nicht dargestellt) kommenden Lichtbündel zu einem Zwischenbild 45 vereinigen, und
- eine optische Baugruppe 46, die das Zwischenbild 45 über die Einkoppeloptik 24 in die Abbildungsstrahlengänge 17.1, 17.2 und damit in die Tubuslinsensysteme 18.1, 18.2 (hier nicht dargestellt) der Beobachtungseinrichtung 13 einblendet.

Die Spiegel 47, 48 und 49 ermöglichen die Anpassung des Strahlenverlaufs an vorgegebene räumliche Verhältnisse.

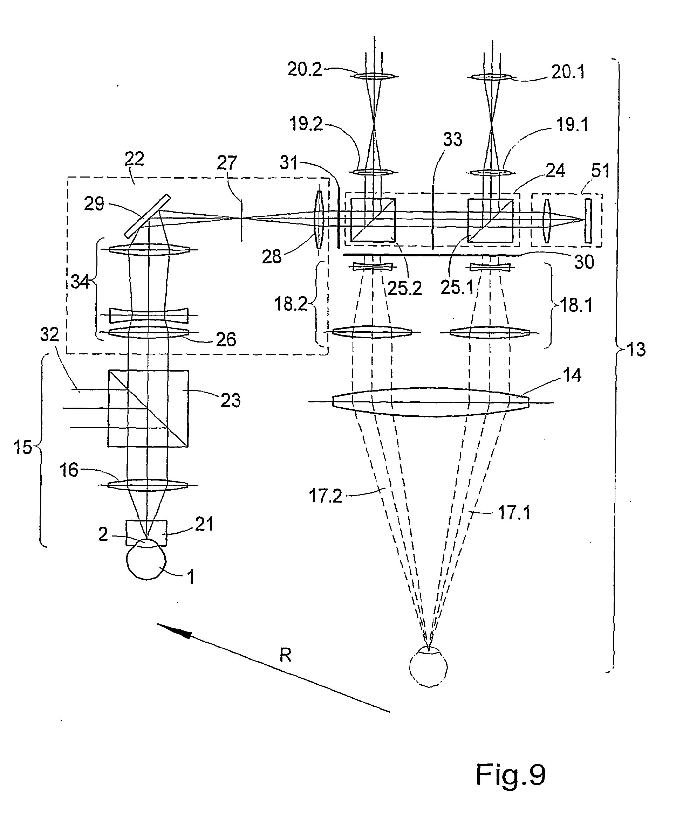

Fig.9 zeigt in Anlehnung an Fig.5 eine Ausgestaltung der erfindungsgemäßen Vorrichtung, bei der die Einkoppeloptik 24 als Binokularteiler ausgebildet ist mit Auskopplung eines auf eine CCD-Kamera 51 gerichteten Teilstrahlengangs.

Mit dieser Ausgestaltung kann sowohl im Vorbereitungs- und Kontrollmodus als auch im Bearbeitungsmodus eine Abbildung des Objektes 1 beziehungsweise des Sektors 2 auf die CCD-Kamera 51 erfolgen. Mit den so gewonnenen Abbildungen kann beispielsweise ein Justiervorgang für den Bearbeitungsmodus unterstützt werden.

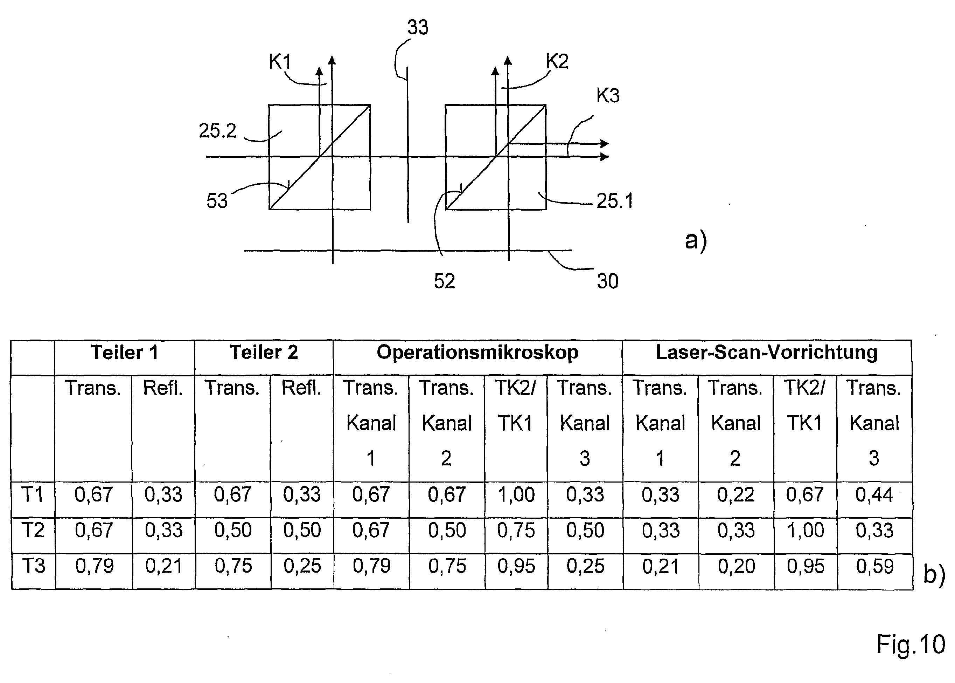

In Fig.10a ist die Einkoppeloptik 24 aus Fig.9 als Einzelheit dargestellt. Hier ist ersichtlich, daß die Verteilung des vom Objekt 1 kommenden Lichts auf drei Kanäle K1, K2, K3 erfolgt, von denen die Kanäle K1, K2 den Tubuslinsensystemen 19.1 bzw. 19.2 (vgl. Fig.9) und der Kanal K3 der CCD-Kamera 51 zugeordnet sind.

Durch geeignete Wahl der Teilungsverhältnisse (Transmission/Reflexion) an den Teilerschichten 52, 53 der Strahlteiler 25.1 bzw. 25.2 können die Helligkeiten der Abbildungen optimiert werden, wobei die optimale Helligkeit der im Vorbereitungs- und Kontrollmodus vom Objekt 1 gewonnenen Abbildungen von der im Bearbeitungsmodus gewünschten Helligkeit verschieden sein kann.

Diesbezüglich sind in der Tabelle nach Fig.10b beispielhaft drei bevorzugte Vorgaben T1, T2, T3 für Teilungsverhältnisse angegeben.

Mit der Vorgabe T1 werden im Vorbereitungs- und Kontrollmodus (über das Objektiv 14 bzw. 16) Abbildungen mit optimierter Helligkeit erzielt. Die Transmission ist hierbei relativ groß, und die Bilder, die über die Kanäle K1 und K2 erzeugt werden, haben untereinander gleiche Helligkeit.

Die Helligkeitswerte der Abbildungen, die im Bearbeitungsmodus (über das Objektiv 16) gewonnen werden, weichen davon ab. Vorteilhaft können auf diese Weise zwei Strahlteiler 25.1 und 25.2 mit gleichen Teilungsverhältnissen verwendet werden.

Mit der Vorgabe T2 sind die Bilder, die im Bearbeitungsmodus gewonnen werden, untereinander gleich hell. Bei der Vorgabe T3 werden im Vorbereitungs- und Kontrollmodus Abbildungen mit gleicher Helligkeit erzielt wie im Bearbeitungsmodus. An den Strahlteiler 25.1 bzw. 25.2 sind hierzu jedoch verschiedene Teilungsverhältnisse notwendig.

Abhängig davon, welche dieser Optionen gewünscht wird, fällt die Vorgabe der Teilungsverhältnisse aus. An dieser Stelle sei vorsorglich darauf hingewiesen, daß in den angegebenen Beispielen von verlustarmen bzw. verlustfreien Schichten ausgegangen wurde. Erreicht die Absorption der Teilerschichten eine relevante Größe, ändern sich die Teilungsverhältnisse im Detail etwas.

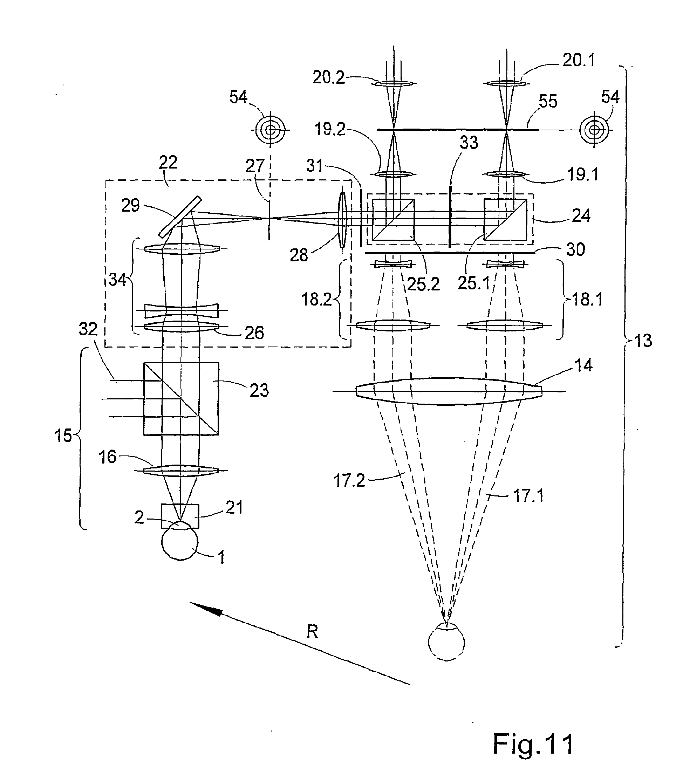

Beim Betreiben der erfindungsgemäßen Vorrichtung ist es zur Vorbereitung der Bearbeitung des Objektes 1 erforderlich, das Objekt 1 relativ zu der Laser-Scan-Einrichtung 15 zu positionieren. Um hierzu dem Bedienungspersonal den Vergleich der Soll-Lage mit der Ist-Lage zu ermöglichen, ist es von Vorteil, die Lage des Objektes 1 relativ zu einem Bezugspunkt am Gerät zu visualisieren. Ein solcher Bezugspunkt kann durch eine Zielmarke 54 geschaffen werden, die auf eine in einer Zwischenbildebene angeordnete transparente Platte aufgebracht ist. Geeignete Zwischenbildlagen sind beispielhaft in Fig.11 dargestellt. Danach kann die mit der Zielmarke 54 versehene Platte im Zwischenbild 27 der Koppeleinrichtung 22 oder auch in den Zwischenbildern 55 der Okulare 20.1, 20.2 stehen.

Darüber hinaus kann der Bezugspunkt auch über ein Display 38 (vgl. Fig.7) visualisiert werden. Zur Visualisierung des Objektes 1 sind verschiedene Realisierungen denkbar. Manche Objekte 1 weisen eine Struktur auf, in der charakteristische Punkte beschreibbar sind. So kann bei einem Patientenauge die Pupillenmitte als Bezug gewählt werden. Eine andere Möglichkeit kann auch das Anbringen einer künstlichen Markierung sein.

Optional kommt die Prozessparametererfassung im Bearbeitungsmodus zum Einsatz, nachdem das zu bearbeitende Objekt 1 relativ zu der Laser-Scan-Einrichtung 15 positioniert ist. So kann beispielsweise das Prinzip der konfokalen Mikroskopie zur Bestimmung der Corneadicke genutzt werden, wenn es sich beim Objekt 1 um ein Auge handelt (Pachymetrie). Auch andere Parameter, wie z.B. die Epitheldicke, sind mit einer solchen Anordnung bestimmbar.
Eine weitere Möglichkeit zur Erfassung von Prozeßparametern besteht in der Verwendung interferometrischer Detektoren. So kann zum Beispiel mit Hilfe von interferometrischen Anordnungen eine OCT (Optical Coherence Tomography) durchgeführt werden.

### Bezugszeichenliste

- 1: Objekt
- 2: Sektor
- 3: Beobachtungseinrichtung
- 4: Objektiv
- 5: Strahlengang
- 5.1, 5.2: Teilstrahlengänge
- 6: Strahlteiler
- 7: Teilersystem
- 8: Laserstrahlung
- 9.1, 9.2: Tubuslinsen
- 10.1, 10.2: Okulare
- 11: Linsengruppe
- 13: Beobachtungseinrichtung
- 14: Objektiv
- 15: Laser-Scann-Einrichtung
- 16: Objektiv
- 17.1, 17.2: Abbildungsstrahlengänge
- 18.1, 18.2: Vergrößerungswechsler
- 19.1, 19.2: Tubuslinsensystem
- 20.1, 20.2: Okulare
- 21: Kontaktglas
- 22: Koppeleinrichtung
- 23: Auskoppeloptik
- 24: Einkoppeloptik
- 25.1, 25.2: Strahlteiler
- 26: Linsengruppe
- 27: Zwischenbild
- 28: Linsengruppe
- 29: Umlenkelement
- 30, 31: Shutter
- 32: Laserstrahlung
- 33: Shutter
- 34: optisches System
- 35: Videoobjektiv
- 36: CCD-Empfänger
- 37: Bildsignalverarbeitungseinrichtung
- 38: Bildwiedergabeeinrichtung
- 39: Linsengruppe
- 40, 41: Frontobjektiv
- 42: Signalweg
- 43: optische Baugruppe
- 44: Feldlinse
- 45: Zwischenbild
- 46: optische Baugruppe
- 47, 48, 49: Spiegel
- 50: Erfassungseinrichtung
- 51: CCD-Kamera
- 52, 53: Teilerschichten
- 54: Zielmarke
- 55: Zwischenbild

- K1, K2, K3: Kanäle
- T1, T2, T3: Vorgaben für Teilungsverhältnisse

- R: Richtung

## Patentansprüche

1. Vorrichtung zur Bearbeitung eines Objektes (1) mit Hilfe von Laserstrahlung (32), im wesentlichen umfassend
- eine Laserquelle, die eine Laserstrahlung (32) mit Pulsen einer Länge im Femtosekundenbereich erzeugt,
- eine der Laserquelle nachgeordnete Laser-Scan-Einrichtung, mit welcher die Laserstrahlung (32) scannend durch einen Sektor (2) innerhalb des Objektes (1) geführt wird, und
**gekennzeichnet durch:**
- eine Beobachtungseinrichtung (13) mit einem ersten Objektiv (14) zur Abbildung des Objektes (1) mit unterschiedlichen Vergrößerungsmaßstäben und
- ein zweites Objektiv (16), **durch** welches hindurch die Laserstrahlung (32) scannend **durch** den Sektor (2) innerhalb des Objektes (1) geführt wird, der bearbeitet werden soll, wobei
- die beiden Objektive (14,16) hinsichtlich der Größe der in den Abbildungen darstellbaren Bereiche und/oder hinsichtlich ihrer Schnittweite voneinander verschieden sind und
- Mittel zur wechselnden Abbildung des Objektes (1) in einem ersten Betriebsmodus **durch** das erste Objektiv (14) und in einem zweiten Betriebsmodus **durch** das zweite Objektiv (16) vorhanden sind,
- wobei die Abbildung des Objektes (1) im zweiten Betriebsmodus gleichzeitig mit der Bearbeitung des Objektes (1) vorgesehen ist.

2. Vorrichtung nach Anspruch 1, **dadurch gekennzeichnet, daß** eine Positioniereinrichtung zur Veränderung der Position des Objektes (1) relativ zu den beiden Objektiven (14,16) vorhanden ist, wobei sich
- im ersten Betriebsmodus das Objekt (1) im Fokus des ersten Objektivs (14) und
- im zweiten Betriebsmodus das Objekt (1) im Fokus des zweiten Objektivs (16) befindet.

3. Vorrichtung nach Anspruch 1 oder 2, **dadurch gekennzeichnet, daß**
- im Strahlengang der Laser-Scan-Einrichtung (15) eine bevorzugt als Strahlteiler ausgeführte Auskoppeloptik (23) für das vom Sektor (2) des Objekts (1) kommende und durch das zweite Objektiv (16) hindurchtretende Licht vorhanden ist,
- im Strahlengang der Beobachtungseinrichtung (13) eine Einkoppeloptik (24) für das ausgekoppelte Licht vorgesehen ist, und
- optische oder opto-elektronische Baugruppen zur Übertragung des Lichts von der Auskoppeloptik (23) zur Einkoppeloptik (24) vorhanden sind.

4. Vorrichtung nach Anspruch 3, **dadurch gekennzeichnet, daß**
- im Lichtweg zwischen dem ersten Objektiv (14) und der Einkoppeloptik (24) ein Shutter (30), vorgesehen ist, der im zweiten Betriebsmodus das vom ersten Objektiv (14) kommende Licht abblockt, und/oder
- im Lichtweg zwischen dem zweiten Objektiv (16) und der Einkoppeloptik (24) ein Shutter (31), vorgesehen ist, der im ersten Betriebsmodus das durch das zweite Objektiv (16) kommende Licht abblockt.

5. Vorrichtung nach einem der vorgenannten Ansprüche, **dadurch gekennzeichnet, daß** die Beobachtungseinrichtung (13) als Stereomikroskop ausgebildet ist und im wesentlichen umfaßt
- das erste Objektiv (14),
- zwei getrennte, eine stereoskopische Darstellung ermöglichende Abbildungsstrahlengänge (17.1, 17.2), und in den beiden Abbildungsstrahlengängen (17.1, 17.2) jeweils
- einen Vergrößerungswechsler (18.1, 18.2) zur Vorgabe unterschiedlicher Vergrößerungen während des ersten Betriebsmodus,
- ein Tubuslinsensystem (19.1, 19.2),
- ein Okular (20.1, 20.2), sowie
- eine aus zwei Strahlteilern (25.1, 25.2) gebildete Einkoppeloptik (24), wobei ein Strahlteiler (25.1) im Abbildungsstrahlengang (17.1) und der zweite Strahlteiler (25.2) im Abbildungsstrahlengang (17.2), bevorzugt jeweils zwischen dem Vergrößerungswechsler (18.1, 18.2) und dem Tubuslinsensystem (19.1, 19.2), eingeordnet ist.

6. Vorrichtung nach Anspruch 5, **dadurch gekennzeichnet, daß** zwischen den beiden Strahlteilern (25.1, 25.2) ein Shutter (33) vorgesehen ist, der eine gegenseitige Beeinflussung der Abbildungsstrahlengänge (17.1, 17.2) im ersten Betriebsmodus verhindert.

7. Vorrichtung nach einem der vorgenannten Ansprüche, **dadurch gekennzeichnet, daß** zur Übertragung des Lichts von der Laser-Scan-Einrichtung (15) zur Beobachtungseinrichtung (13) optische Baugruppen aus Linsen, Prismen, Strahlumlenkelementen und/oder faseroptische Lichtleiter vorgesehen sind.

8. Vorrichtung nach Anspruch 7, **dadurch gekennzeichnet, daß** zur Übertragung des Lichts von der Laser-Scan-Einrichtung (15) zur Beobachtungseinrichtung (13) optische Baugruppen vorgesehen sind, im wesentlichen umfassend
- eine Linsengruppe (26) zur Vereinigung der durch das zweite Objektiv (16) kommenden Lichtbündel zu einem Zwischenbild (27),
- eine Linsengruppe (28) zur Abbildung des Zwischenbildes (27) in den Abbildungsstrahlengang der Beobachtungseinrichtung (13) sowie
- optische Elemente zur Strahlumlenkung und/oder Strahlfaltung.

9. Vorrichtung nach einem der Ansprüche 7 oder 8, **dadurch gekennzeichnet, daß** in den Übertragungsweg des Lichtes ein optisches System (34) zur Variation der Brennweite und damit des Abbildungsmaßstabes eingeordnet ist, bevorzugt mit zwei in ihrer Position zueinander veränderbaren Linsen oder Linsengruppen.

10. Vorrichtung nach einem der Ansprüche 1 bis 6, **dadurch gekennzeichnet, daß** zur Übertragung des Lichts von der Laser-Scan-Einrichtung (15) zur Beobachtungseinrichtung (13) opto-elektronische Baugruppen vorgesehen sind, wobei
- im zweiten Betriebsmodus die Abbildung des Objektes (1) mittels des zweiten Objektivs (16) über ein Videoobjektiv (35) auf einen CCD-Empfänger (36) erfolgt,
- dem CCD-Empfänger (36) eine Bildsignalverarbeitungseinrichtung (37) und eine Bildwiedergabeeinrichtung (38) nachgeordnet sind und
- Mittel zur Einkopplung des auf der Bildwiedergabeeinrichtung (38) wiedergegebenen Bildes in die Abbildungstrahlengänge (17.1, 17.2) der Beobachtungseinrichtung (13) vorhanden sind, so daß
- das im zweiten Betriebsmodus mittels des zweiten Objektivs (16) erzeugte Bild durch die Okulare (20.1, 20.2) hindurch sichtbar ist.

11. Vorrichtung nach Anspruch 10, **dadurch gekennzeichnet, daß** als Bildwiedergabeeinrichtung (38) ein LC-Display vorgesehen ist.

12. Vorrichtung nach einem der vorgenannten Ansprüche, **dadurch gekennzeichnet, daß** zur Übertragung des Lichts von der Laser-Scan-Einrichtung (15) zur Beobachtungseinrichtung (13) sowohl optische Baugruppen als auch opto-elektronische Baugruppen verfügbar und wahlweise gegeneinander austauschbar sind.

13. Vorrichtung nach einem der vorgenannten Ansprüche, **dadurch gekennzeichnet, daß** eine Erfassungseinrichtung (50) vorhanden ist zur Erfassung von
- den Vorgang der Bearbeitung betreffenden Prozeßparametern,
- die Eigenschaften des Materials, aus dem das Objekt (1) besteht, betreffenden Werten, oder
- die Abmessungen des Objektes (1) betreffenden Werten.

14. Vorrichtung nach Anspruch 13, **dadurch gekennzeichnet, daß** als Beobachtungseinrichtung (13) ein Stereomikroskop nach dem Greenough-Typ vorgesehen ist mit zwei Frontobjektiven (40, 41), von denen jeweils eines einem Abbildungsstrahlengang (17.1, 17.2) zugeordnet ist, und die Erfassungseinrichtung (50) im wesentlichen im Raum zwischen den beiden Frontobjektiven (40, 41) angeordnet ist.

15. Vorrichtung nach einem der vorgenannten Ansprüche, **dadurch gekennzeichnet, daß** Mittel zur Einspiegelung von
- den Vorgang der Bearbeitung betreffenden Prozeßparametern,
- die Eigenschaften des Materials, aus dem das Objekt (1) besteht, betreffenden Werten, oder
- die Abmessungen des Objektes (1) betreffenden Werten in einen der oder beide Abbildungsstrahlengänge (17.1, 17.2) der Beobachtungseinrichtung (13) vorgesehen sind.

16. Vorrichtung nach einem der vorgenannten Ansprüche, **dadurch gekennzeichnet, daß** Mittel zur Auskopplung eines Teilstrahlengangs aus dem vom Objekt (1) kommenden und durch die Objektive (14, 16) hindurchtretenden Lichts vorgesehen und dieser zwecks Abbildung des Objektes (1) auf eine CCD-Kamera (51) gerichtet ist.

17. Vorrichtung nach einem der vorgenannten Ansprüche, **dadurch gekennzeichnet, daß** Mittel zur Visualisierung der Lage des Objektes (1) relativ zu einem Bezugspunkt vorhanden sind, wobei der Bezugspunkt bevorzugt in Form einer Zielmarke (54) ausgebildet ist.

18. Vorrichtung nach Anspruch 17, **dadurch gekennzeichnet, daß** die Zielmarke (54) auf einer optischen Platte fixiert und diese im Zwischenbild eines Abbildungsstrahlengangs positioniert ist.

## Claims

1. Apparatus for processing an object (1) with the aid of laser radiation (32), substantially comprising
- a laser source, which generates a laser radiation (32) with pulses having a length in the femtoseconds range,
- a laser scanning device disposed downstream of the laser source, by means of which laser scanning device the laser radiation (32) is guided in scanning fashion through a sector (2) within the object (1), and
**characterized by:**
- an observation device (13) having a first objective (14) for imaging the object (1) with different magnification scales, and
- a second objective (16), through which the laser radiation (32) is guided in scanning fashion through that sector (2) within the object (1) which is intended to be processed, wherein
- the two objectives (14, 16) differ from one another with regard to the size of the regions that can be represented in the images and/or with regard to their vertex focal length, and
- means for the changing imaging of the object (1) in a first operating mode by means of the first objective (14) and in a second operating mode by means of the second objective (16) are present,
- wherein the imaging of the object (1) in the second operating mode is provided at the same time as the processing of the object (1).

2. Apparatus according to Claim 1, **characterized in that** a positioning device for altering the position of the object (1) relative to the two objectives (14, 16) is present, wherein
- in the first operating mode the object (1) is situated at the focus of the first objective (14), and
- in the second operating mode the object (1) is situated at the focus of the second objective (16).

3. Apparatus according to Claim 1 or 2, **characterized in that**
- a coupling-out optical unit (23), preferably embodied as a beam splitter, for the light that comes from the sector (2) of the object (1) and passes through the second objective (16) is present in the beam path of the laser scanning device (15),
- a coupling-in optical unit (24) for the coupled-out light is provided in the beam path of the observation device (13), and
- optical or optoelectronic assemblies for transmitting the light from the coupling-out optical unit (23) to the coupling-in optical unit (24) are present.

4. Apparatus according to Claim 3, **characterized in that**
- a shutter (30) is provided in the light path between the first objective (14) and the coupling-in optical unit (24), said shutter blocking the light coming from the first objective (14) in the second operating mode, and/or
- a shutter (31) is provided in the light path between the second objective (16) and the coupling-in optical unit (24), said shutter blocking the light coming through the second objective (16) in the first operating mode.

5. Apparatus according to any of the preceding claims, **characterized in that** the observation device (13) is embodied as a stereomicroscope and substantially comprises
- the first objective (14),
- two separate imaging beam paths (17.1, 17.2) that enable a stereoscopic representation, and in the two imaging beam paths (17.1, 17.2) in each case
- a magnification changer (18.1, 18.2) for predefining different magnifications during the first operating mode,
- a tube lens system (19.1, 19.2),
- an eyepiece (20.1, 20.2), and also
- a coupling-in optical unit (24) formed from two beam splitters (25.1, 25.2), wherein one beam splitter (25.1) is arranged in the imaging beam path (17.1) and the second beam splitter (25.2) is arranged in the imaging beam path (17.2), preferably in each case between the magnification changer (18.1, 18.2) and the tube lens system (19.1, 19.2).

6. Apparatus according to Claim 5, **characterized in that** a shutter (33) is provided between the two beam splitters (25.1, 25.2), said shutter preventing the imaging beam paths (17.1, 17.2) from influencing one another in the first operating mode.

7. Apparatus according to any of the preceding claims, **characterized in that** optical assemblies composed of lenses, prisms, beam deflection elements and/or fibre-optic waveguides are provided for transmitting the light from the laser scanning device (15) to the observation device (13).

8. Apparatus according to Claim 7, **characterized in that** optical assemblies are provided for transmitting the light from the laser scanning device (15) to the observation device (13), said optical assemblies substantially comprising
- a lens group (26) for combining the light bundles coming through the second objective (16) to form an intermediate image (27),
- a lens group (28) for imaging the intermediate image (27) into the imaging beam path of the observation device (13), and
- optical elements for beam deflection and/or beam folding.

9. Apparatus according to either of Claims 7 and 8, **characterized in that** an optical system (34) for varying the focal length and thus the imaging scale is arranged into the light transmission path, preferably with two lenses or lens groups that can be altered in terms of their position with respect to one another.

10. Apparatus according to any of Claims 1 to 6, **characterized in that** optoelectronic assemblies are provided for transmitting the light from the laser scanning device (15) to the observation device (13), wherein
- in the second operating mode the object (1) is imaged by means of the second objective (16) via a video objective (35) onto a CCD receiver (36),
- an image signal processing device (37) and an image reproduction device (38) are disposed downstream of the CCD receiver (36), and
- means for coupling the image reproduced on the image reproduction device (38) into the imaging beam paths (17.1, 17.2) of the observation device (13) are present, such that
- the image generated by means of the second objective (16) in the second operating mode is visible through the eyepieces (20.1, 20.2).

11. Apparatus according to Claim 10, **characterized in that** an LC display is provided as the image reproduction device (38).

12. Apparatus according to any of the preceding claims, **characterized in that** both optical assemblies and optoelectronic assemblies are available for transmitting the light from the laser scanning device (15) to the observation device (13) and are optionally mutually interchangeable.

13. Apparatus according to any of the preceding claims, **characterized in that** a detection device (50) is present for detecting
- process parameters concerning the processing operation,
- values concerning the properties of the material of which the object (1) consists, or
- values concerning the dimensions of the object (1).

14. Apparatus according to Claim 13, **characterized in that** a stereomicroscope according to the Greenough type is provided as the observation device (13), comprising two front objectives (40, 41), each of which is respectively assigned to an imaging beam path (17.1, 17.2), and the detection device (50) is disposed substantially in the space between the two front objectives (40, 41).

15. Apparatus according to any of the preceding claims, **characterized in that** provision is made of means for reflecting
- process parameters concerning the processing operation,
- values concerning the properties of the material of which the object (1) consists, or
- values concerning the dimensions of the object (1), into one of the or both imaging beam paths (17.1, 17.2) of the observation device (13).

16. Apparatus according to any of the preceding claims, **characterized in that** means for coupling out a partial beam path from the light that comes from the object (1) and passes through the objectives (14, 16) are provided and said partial beam path is directed onto a CCD camera (51) for the purpose of imaging the object (1).

17. Apparatus according to any of the preceding claims, **characterized in that** means for visualizing the position of the object (1) relative to a reference point are present, wherein the reference point is preferably embodied in the form of a target mark (54).

18. Apparatus according to Claim 17, **characterized in that** the target mark (54) is fixed on an optical plate and the latter is positioned in the intermediate image of an imaging beam path.

## Revendications

1. Dispositif pour usiner un objet (1) à l'aide de rayonnement laser (32) comportant pour l'essentiel
- une source laser générant un rayonnement laser (32) avec des impulsions dont la largeur se situe dans la plage des fentosecondes,
- un appareillage de balayage laser en aval de la source laser permettant de piloter le rayonnement laser (32) pour balayer un secteur (2) à l'intérieur d'un objet (1), et
**caractérisé en ce que**
- un appareillage d'observation (13) avec un premier objectif (14) pour la représentation de l'objet (1) avec diverses échelles de grossissements et
- un deuxième objectif (16) à travers lequel on pilote le balayage du rayonnement laser (32) sur le secteur (2) à l'intérieur de l'objet (1) à usiner, dans lequel
- les deux objectifs (14, 16) sont différents l'un de l'autre du point de vue de la taille des zones pouvant être visualisées sur la représentation et/ou du point de vue de leur tirage, et
- on dispose de moyens pour la représentation alternative de l'objet (1) dans un premier mode de fonctionnement à travers le premier objectif (14) et dans un deuxième mode de fonctionnement à travers le deuxième objectif (16),
- la représentation de l'objet (1) dans le deuxième mode de fonctionnement étant prévue simultanément à l'usinage de l'objet (1).

2. Dispositif selon la revendication 1, **caractérisé en ce qu'**on dispose d'un appareillage de positionnement pour modifier la position de l'objet (1) par rapport aux deux objectifs (14, 16), dans lequel
- l'objet (1) se trouve au foyer du premier objectif (14) dans le premier mode de fonctionnement et
- l'objet (1) se trouve au foyer du deuxième objectif (16) dans le deuxième mode de fonctionnement.

3. Dispositif selon la revendication 1 ou 2, **caractérisé en ce que**
- dans le trajet des rayons de l'appareillage de balayage laser (15), on dispose d'une optique de couplage en sortie réalisée de préférence sous la forme de séparateur de faisceaux (23) pour la lumière provenant du secteur (2) de l'objet (1) et traversant le deuxième objectif (16),
- dans le trajet des rayons de l'appareillage d'observation (13), on a prévu une optique d'entrée (24) pour la lumière en sortie, et
- on dispose de modules optiques ou optoélectroniques pour la transmission de la lumière depuis l'optique de couplage en sortie (23) vers l'optique de couplage en entrée (24).

4. Dispositif selon la revendication 3, **caractérisé en ce que**
- un obturateur (30) est prévu dans le chemin optique entre le premier objectif (14) et l'optique de couplage en entrée (24) pour bloquer la lumière provenant du premier objectif (14) dans le deuxième mode de fonctionnement, et/ou
- un obturateur (31) est prévu dans le chemin optique entre le deuxième objectif (16) et l'optique de couplage en entrée (24) pour bloquer la lumière provenant du deuxième objectif (16) dans le premier mode de fonctionnement.

5. Dispositif selon une des revendications précédentes, **caractérisé en ce que** l'appareillage d'observation (13) est réalisé sous forme de microscope stéréoscopique et comporte pour l'essentiel
- le premier objectif (14),
- deux trajets de rayons de reproduction séparés (17.1, 17.2) permettant une représentation stéréoscopique, et dans les deux trajets de rayons de reproduction (17.1, 17.2), respectivement
- un changeur de grossissement (18.1, 18.2) pour permettre divers grossissements dans le premier mode de fonctionnement,
- un système de lentilles des tubes (19.1, 19.2)
- un oculaire (20.1, 20.2), ainsi que
- une optique de couplage en entrée (24) constituée de deux séparateurs de faisceaux (25.1, 25.2), un séparateur de faisceaux (25.1) étant disposé dans le trajet des rayons de reproduction (17.1) et le deuxième séparateur de faisceaux (25.2) étant disposé dans le trajet des rayons de reproduction (17.2), de préférence respectivement entre le changeur de grossissement (18.1, 18.2) et le système de lentilles des tubes (19.1, 19.2).

6. Dispositif selon la revendication 5, **caractérisé en ce qu'**un obturateur (33) est prévu entre les deux séparateurs de faisceaux (25.1, 25.2) pour éviter une influence mutuelle des trajets de rayons de reproduction (17.1, 17.2) dans le premier mode de fonctionnement.

7. Dispositif selon une des revendications précédentes, **caractérisé en ce que** des modules optiques de lentilles, de prismes, d'éléments de déviation du rayonnement et/ou des conducteurs de lumière en fibres optiques sont prévus pour la transmission de la lumière depuis l'appareillage de balayage laser (15) vers appareillage d'observation (13).

8. Dispositif selon la revendication 7, **caractérisé en ce que** des modules optiques sont prévus pour la transmission de la lumière depuis l'appareillage de balayage laser (15) vers appareillage d'observation (13), comportant pour l'essentiel
- un groupe de lentilles (26) pour la convergence du faisceau lumineux provenant à travers le deuxième objectif (16) dans une image intermédiaire (27),
- un groupe de lentilles (28) pour la reproduction de l'image intermédiaire (27) dans le trajet des rayons de reproduction de l'appareillage d'observation (13) ainsi que
- des éléments optiques pour la déviation de rayons et/ou le repliement de rayons.

9. Dispositif selon une des revendications 7 ou 8, **caractérisé en ce qu'**un système optique (34) pour la variation de la distance focale et ainsi de l'échelle de la reproduction est disposé dans le chemin de transmission de la lumière, de préférence avec deux lentilles ou groupes de lentilles dont on peut modifier la position mutuelle.

10. Dispositif selon une des revendications 1 à 6, **caractérisé en ce que** des modules optoélectroniques sont prévus pour la transmission de la lumière depuis l'appareillage de balayage laser (15) vers l'appareillage d'observation (13), dans lesquels
- la reproduction de l'objet (1) est réalisée au moyen du deuxième objectif (16) par l'intermédiaire d'un objectif vidéo (35) sur un capteur CCD (36) dans le deuxième mode de fonctionnement et
- un appareillage de traitement de signal d'image (37) et un appareillage de restitution de l'image (38) sont disposés en aval du capteur CCD (36),
- on dispose de moyens pour coupler en entrée l'image restituée sur l'appareillage de restitution de l'image (38) vers les trajets de rayons de reproduction (17.1, 17.2) de l'appareillage d'observation (13), de telle sorte que
- l'image générée au moyen du deuxième objectif (16) est visible à travers les oculaires (20.1, 20.2) dans le deuxième mode de fonctionnement.

11. Dispositif selon la revendication 10, **caractérisé en ce qu'**un écran LCD est prévu comme appareillage de restitution de l'image (38).

12. Dispositif selon une des revendications précédentes, **caractérisé en ce qu'**on dispose de modules optiques aussi bien qu'optoélectroniques qu'on peut au choix échanger mutuellement, pour la transmission de la lumière depuis l'appareillage de balayage laser (15) vers l'appareillage d'observation (13).

13. Dispositif selon une des revendications précédentes, **caractérisé en ce qu'**on dispose d'un appareillage de saisie (50) pour la saisie
- de paramètres du processus concernant le déroulement de l'usinage
- de valeurs concernant les caractéristiques du matériau constituant l'objet (1), ou
- des valeurs concernant les dimensions de l'objet (1).

14. Dispositif selon la revendication 13, **caractérisé en ce qu'**un microscope stéréoscopique du type Greenough est prévu comme appareillage d'observation (13) avec deux objectifs frontaux (40, 41) dont respectivement un est attribué à un trajet de rayons de reproduction (17.1, 17.2), et l'appareillage de saisie (50) étant pour l'essentiel disposé dans l'espace entre les deux objectifs frontaux (40, 41).

15. Dispositif selon une des revendications précédentes, **caractérisé en ce qu'**on a prévu dans un ou dans les deux trajets de rayons de reproduction (17.1, 17.2) de l'appareillage d'observation (13), des moyens pour l'incrustation
- de paramètres du processus concernant le déroulement de l'usinage
- de valeurs concernant les caractéristiques du matériau constituant l'objet (1), ou
- des valeurs concernant les dimensions de l'objet (1).

16. Dispositif selon une des revendications précédentes, **caractérisé en ce qu'**on a prévu des moyens pour coupler vers l'extérieur un trajet de rayons partiel d'une lumière provenant de l'objet (1) et traversant les objectifs (14, 16) et qu'on le dirige sur une caméra CCD (51) en vue de la reproduction de l'objet (1).

17. Dispositif selon une des revendications précédentes, **caractérisé en ce qu'**on dispose de moyens pour la visualisation de la position de l'objet (1) par rapport à un point de référence, le point de référence étant de préférence réalisé sous la forme d'une marque de visée (54).

18. Dispositif selon la revendication 17, **caractérisé en ce que** la marque de visée (54) est fixée sur une plaque optique et que celle-ci est positionnée dans l'image intermédiaire d'un trajet de rayons de reproduction.
